# EUROPEAN PATENT SPECIFICATION

(11) **EP 4 525 823 B1**
(45) Date of publication and mention of the grant of the patent: **21.01.2026**
(21) Application number: 23729018.4
(22) Date of filing: 18.05.2023
(51) Int. Cl.: A61K 8/37, A61Q 5/00, A61Q 7/00

(54) **OLFACTORY RECEPTOR 2A4/7 AGONIST FOR PROMOTING HAIR GROWTH AND IMPROVING AESTHETIC ASPECT**
OLFAKTORISCHER REZEPTOR 2A4/7-AGONIST ZUR FÖRDERUNG DES HAARWUCHSES UND ZUR VERBESSERUNG DES ÄSTHETISCHEN AUSSEHENS
AGONISTE DU RÉCEPTEUR OLFACTIF 2A4/7 POUR FAVORISER LA POUSSE DES CHEVEUX ET AMÉLIORER L'ASPECT ESTHÉTIQUE

(30) Priority: 18.05.2022 IT 202200010358
(43) Date of publication of application: 26.03.2025
(73) Proprietor: Giuliani S.p.A., 20129 Milano (IT)
(72) Inventor: GIULIANI, Giammaria, 6926 MONTAGNOLA (CH); RINALDI, Fabio, 20122 MILANO (IT); PINTO, Daniela, 20151 MILANO (IT); MARZANI, Barbara, 27020 Carbonara Al Ticino (IT)
(74) Representative: Cattaneo, Elisabetta
(86) International application number: PCT/EP2023/063421
(87) International publication number: WO 2023/222847

(56) References cited:
- EP-A1- 2 033 623
- WO-A1-2017/198818
- CHÉRET JÉRÉMY ET AL: "Olfactory receptor OR2AT4 regulates human hair growth", vol. 9, no. 1, 1 December 2018 (2018-12-01), XP055922250, Retrieved from the Internet <URL:https://www.nature.com/articles/s41467-018-05973-0.pdf> DOI: 10.1038/s41467-018-05973-0
- LEE SUNG-JOON ET AL: "Therapeutic potential of ectopic olfactory and taste receptors", NATURE REVIEWS DRUG DISCOVERY, NATURE PUBLISHING GROUP, GB, vol. 18, no. 2, 30 November 2018 (2018-11-30), pages 116 - 138, XP036688583, ISSN: 1474-1776, [retrieved on 20181130], DOI: 10.1038/S41573-018-0002-3
- EDELKAMP J ET AL: "Treatment with cyclohexyl salicylate, an olfactory receptor 2A4/7 agonist, promotes human hair follicle growth and bulge stem cell progeny expansion", 51ST ANNUAL ESDR MEETING, 28 September 2022 (2022-09-28), XP093009802

## Description

### FIELD OF THE INVENTION

The present invention relates to an olfactory receptor 2A4/7 agonist for promoting hair growth and improving an aesthetic aspect of hair.

The present invention originates in the cosmetic and trichological field.

Specifically the present invention concerns with a composition containing an olfactory receptor 2A4/7 agonist as active ingredient either for improving an aesthetic aspect of hair and for treating hair loss of a mammal, especially a human being.

### PRIOR ART

Hair has a protecting role and are considered as a skin annex along with sebaceous glands, sudoriferous glands and nails.

The life cycle of the hair bulb includes three main phases: a growth phase known as anagen, an involution phase known as catagen and a rest phase known as telogen. Hair on the scalp is produced by hair follicle, a mammalian skin organ. Hair follicles do not continuously produce hair. They cycle through a growth stage that can last two or more years and regress to a resting stage for up to two months before starting to grow a new hair fiber again.

The biological phases of this phenomenon reside in the capability of stem cells of the bulb to leave, at alternating steps, their state of quiescence. During the bulb growth and hair production phase the proliferation, differentiation and survival activities are prevailing which are regulated by growth factors. The regression phase, on the contrary, is characterized by the activation of molecular pathways that induce apoptosis in bulb cells.

In the anagen phase, the dermal papilla generates chemical signals that activate and instruct the stem cells of the bulge which by migrating to the base of the follicle form the hair matrix. With this "migration" the stem cells of the bulge create a "path" of cells which will give rise to the outer root sheath or ORS.

In response to further signals by the dermal papilla, the matrix cells, which derive from stem cells, proliferate and start the differentiation process, by moving upwards to form the shaft and inner sheath of the hair follicle.

The start of the catagen phase is characterized by the end of cell proliferation and the apoptosis of matrix cells. During catagen the dermal papilla migrates towards the lowermost portion of the bulge. This close proximity of the papilla to the bulge is believed to be essential for initiating another hair production cycle. This enables interaction/activation of bulge cells at rest and a new cycle of hair growth.

Upon catagen/telogen transition, some cells of the bulge migrate to meet the papilla, generating the hair germ.

Hair in telogen contains a cell population at its base, which is in fact called hair germ, located in close proximity to the dermal papilla. The hair germ is activated to proliferate towards the end of the telogen phase, even before the bulge, to form, by surrounding the papilla, the matrix of the new bulb.

Also eyelashes go through the same three distinct phases during their growth cycle: Anagen Catagen, and Telogen but the growth rate and anagen phase duration are shorter than the ones observed in scalp hair.

Different factors, among which stress, the lack of nutrients and ageing, negatively affect the life cycle of the hair bulb, determining a reduction of the number of hairs and their thinning.

Hair growth disorders are very common in particular in the male population. If the role of hair is considered in social relations, hair loss may be hard to face for many people.

One of the more widely spread hair disorder is represented by alopecia, a disorder in the hair growth which may be caused by different factors from genetics to environmental. The degree and pattern of alopecia may vary, however one of the most common alopecia form is represented by androgenetic alopecia (AGA). Alopecia forms other than androgenetic alopecia include telogen effluvium, alopecia areata, ringworm, scarring alopecia, and hair loss due to cosmetic overprocessing. In individuals affected by androgenetic alopecia (AGA), the follicles that form at the start of the new anagen phase decrease in size with the passing of time

(miniaturisation), leading, as time proceeds, to the formation of a hair that is smaller than the previous one. The result is the formation of a microscopic hair.

Dermatologists classify alopecias by subdividing them into the cicatricial and noncicatricial categories. Some types of alopecia such as lichen planopilaris, discoid lupus erythematous and graft-versus-host-disease are associated with the bulge follicle stem cell destruction and with permanent hair loss. In the reversible types of alopecia such as in alopecia areata, inflammation affects the follicle progenitor cells, but spares the stem cells thereof. In these diseases, regrowth takes place on elimination of the inflammation and on the consequent regeneration of the hair follicle starting from undamaged stem cells.

In individuals suffering from androgenetic alopecia (AGA), over time the follicles, which are formed again at the beginning of the new anagen phase, become smaller in size, leading to the formation of hair with smaller diameter as compared to the initial diameter. As a consequence, the formation of microscopic hair occurs.

It was observed that although the follicles of scalp atrophy, there still remains a supply of stem cells which convert to progenitor cells, even though to a lesser extent as compared to the scalp in physiological conditions.

At present many options for the treatment of hair growth disorders, included alopecia, are available.

A first line of treatments includes the topical application and/or the administration of formulations for preventing and treating hair growth disorders. Most of the hair formulations available on the market target hair bulbs and act on scalp metabolism, feeding, oxygenation and microcirculation improving the conditions contributing to a physiological growth of hair.

Typically, these trichological products incorporate cosmetic ingredients for restoring skin and the conditions suitable for promoting hair growth.

A large number of trichological products contain antioxidant substances, such as vitamin E, glycosaminoglycan such as hyaluronic acid, and vitamins such as vitamin A and vitamins of the B group or derivatives thereof such as niacin or nicotinamide adenine dinucleotide (NAD).

However, in many cases the application of trichological compositions containing the above and other ingredients to the scalp or the administration of nutritional formulations have shown a poor effect on the stimulation of hair growth.

Trichological products containing pharmacologically active substances as trichological ingredients have shown a superior activity with respect to the cosmetic/trichological formulations.

Amongst these pharmaceutical products, the formulations containing minoxidil, a pharmacologically active ingredient provided with vasodilator activity, are well known. These products are usually formulated as alcoholic solutions for topical application.

Other products used for treating hair disorders include pharmaceutical compositions containing finasteride, an hormonal substance. These products inhibit the Type II 5-alpha-reductase, an enzyme converting testosterone into dihydrotestosterone (DHT), a hormone which shrinks or miniaturizes hair follicles and leads to baldness when the secretion exceeds the physiological production.

However, the use of minoxidil and finasteride are not free from drawbacks. Specifically, the topical application of minoxidil may lead to local side effects such as skin rashes, local inflammations, and to general drawback such as headache, hirsutism, while the oral administration of finasteride may results in hormonal dysfunctions.

WO2017/198818 describes the cosmetic use, as well as the therapeutic use in promoting hair growth or treatment of hair loss, of compounds as sandal pentanol and sandal cyclopentane. These compounds can be incorporated e.g. in a styling gel, hair conditioner or shampoo, and are shown to stimulate the olfactory receptor OR2AT4, which is expressed in human scalp hair follicles.

There is a need for alternative remedies suitable in the treatment of both aesthetic problems of hair and hair growth disorders whose topical use is effective and substantially free of side effects.

One of the aims of the present invention consists in providing new uses in the trichological field for an olfactory receptor 2A4/7 agonist.

Another object of the invention is to provide a composition for the cosmetic treatment either of aesthetic problems of hair and hair growth disorders.

### SUMMARY OF THE INVENTION

The scope of this invention is defined by the claims. Any references in the description to methods of treatment refer to the compounds and pharmaceutical compositions of the present invention for use in a method for treatment of the huma (or animal) body by therapy.

In accordance with a general aspect, the present invention provides new uses in the trichological field for a selected olfactory receptor 2A4/7 agonist.

The present invention origins from the study of the role of olfactory receptor OR2A4/7 in human melanocytes and from OR2A4/7 protein expression in the infundibulum, and isthmus, (suprabasal ORS) of HF in normal human skin in situ.

The inventors found that cyclohexyl salicylate (CHS), which is a specific olfactory receptor 2A4/7 agonist, promotes epidermal keratinocyte proliferation and differentiation and exerts an important role in the process governing certain steps of hair growth.

In particular, OR2A4/7 is functionally expressed and active in human melanocytes, because CHS elicits transient intracellular Ca2+ signals.

The herein disclosed selected olfactory receptor 2A4/7 agonist is suitable either for the cosmetic treatment of a population of individuals having aesthetic problems of hair and for the therapeutical use of a population of individuals affected by hair growth disorders or diseases.

Accordingly, in a first aspect the invention provides for the non- therapeutic, cosmetic use of an olfactory receptor 2A4/7 agonist for improving an aesthetic aspect of hair, or for treating an aesthetic deficit of hair, wherein the olfactory receptor 2A4/7 agonist is cyclohexyl salicylate.

In accordance with an embodiment, the cosmetic use of cyclohexyl salicylate for the treatment of hair thinning.

The topical application of cyclohexyl salicylate stimulates the olfactory receptor (OR) 2A4/7 which in turns, prolongs human hair growth and promotes a physiological hair growth.

According to another aspect, the invention concerns with the cosmetic use of a composition comprising an olfactory receptor 2A4/7 agonist and a physiologically acceptable carrier, for improving an aesthetic aspect of the hair wherein the olfactory receptor 2A4/7 agonist is cyclohexyl salicylate.

Typically, the composition is a cosmetic composition for topical application on the skin or scalp.

In accordance with certain embodiments, the composition for cosmetic use is a cosmetic composition containing cyclohexyl salicylate which may be used to treat cosmetic or aesthetic defects or blemish of hair such as hair thickening or discoloration.

The topical application of cyclohexyl salicylate to a subject suffering from hair thinning gives a progressive thickening of thinner areas of the scalp.

According to another aspect, the invention concerns with an olfactory receptor 2A4/7 agonist for use in the prevention or treatment of a hair growth disorder wherein the olfactory receptor 2A4/7 agonist is cyclohexyl salicylate.

Advantageously, the cyclohexyl salicylate is suitable in the treatment of Hair Follicle (HF) disorders and aging resulting in hair loss.

In accordance with another aspect, a pharmaceutical composition is herein provided, comprising an olfactory receptor 2A4/7 agonist and a pharmaceutically acceptable carrier for use in the prevention or treatment of a hair growth disorder wherein the olfactory receptor 2A4/7 agonist is cyclohexyl salicylate.

Preferably, the composition for medical uses is for topical application. The herein disclosed topically applicable compositions are suitable as an alternative or as therapeutic addition for drugs to improve hair density and ameliorating the aesthetic aspect.

According to certain embodiments, the composition of the present invention is a pharmaceutical composition.

Typically, the composition of the invention contains a cosmetically or pharmaceutically active amount of the olfactory receptor 2A4/7 agonist herein disclosed.

The composition as herein described may be applied on the skin or scalp of an individual for the intended use.

Certain advantages and features connected with the cosmetic and medical uses of the olfactory receptor 2A4/7 agonist are further described with reference to the appended Figures whose content is briefly illustrated hereinbelow.

### BRIEF DESCRIPTION OF THE DRAWINGS

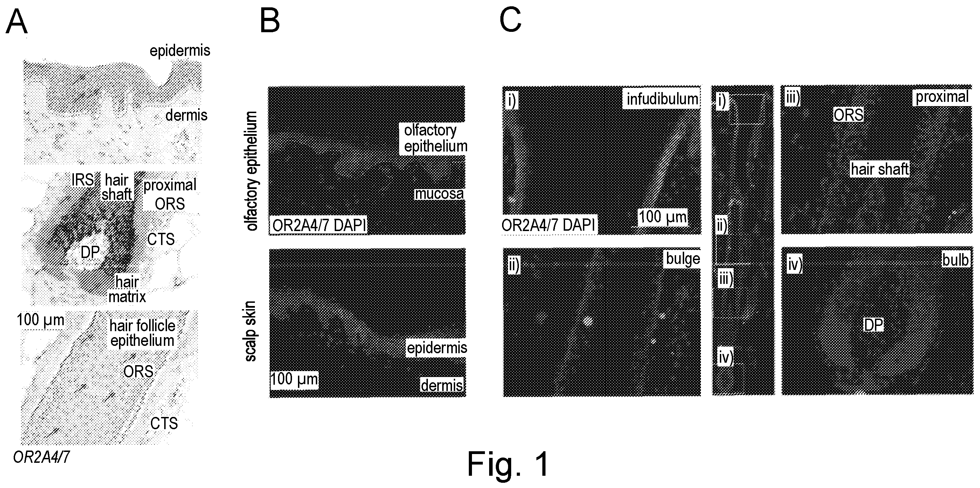

Figure 1. A illustrates images of OR2A4/7 mRNA expression (pink arrows that are dark grey in black and white figure version) in olfactory epithelium (upper panel), hair follicle (HF) bulb (middle panel) and bulge (lower panel). Figure 1 B) illustrates representative images of OR2A4/7 protein expression in olfactory epithelium (positive control; upper panel), and fresh human scalp skin epidermis (lower panel). Figure 1 C) illustrates the different HF compartments in fresh human scalp skin. n=3 independent donors. CTS: connective tissue sheath; DP: dermal papilla; IRS: inner root sheath; ORS: outer root sheath;

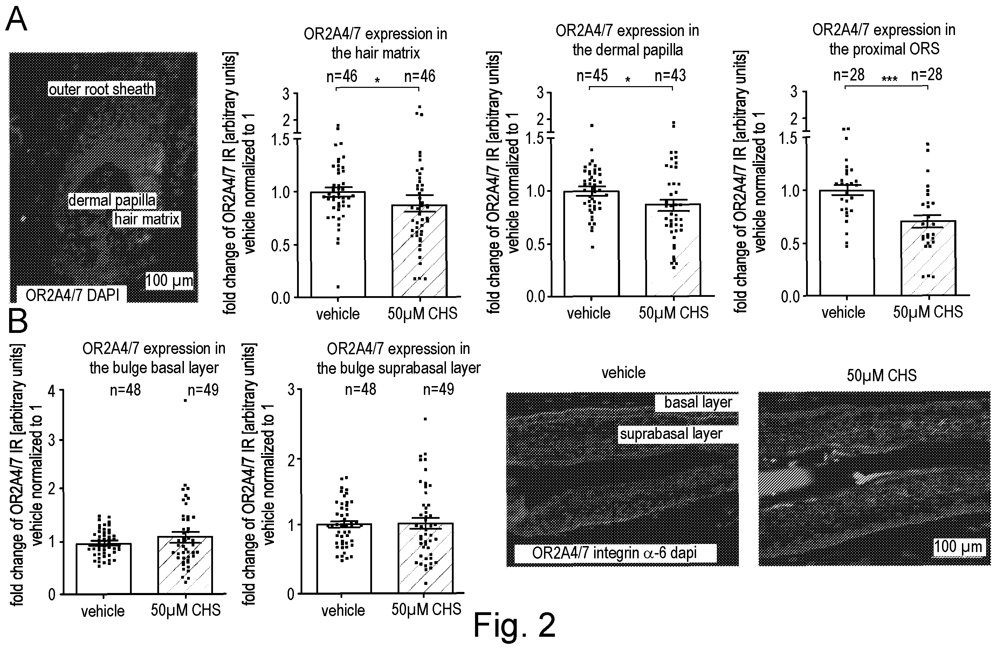

Figure 2. A) shows images of OR2A4/7 (green, light grey in black and white figure version) protein expression in different compartments of the bulb of organ-cultured HFs ex vivo (left panel); bar graphs showing quantitative analysis of pooled data on OR2A4/7 expression in the dermal papilla, hair matrix (germinative + precortical), and outer root sheath under treatment with vehicle or 50µM CHS (right panel).
Figure 2 B) illustrates bar graphs showing quantitative analysis of pooled data on OR2A4/7 expression in the bulge basal and suprabasal layers under treatment with vehicle or 50µM CHS (left panel); representative images of the bulge from vehicle and 50µM CHS treated HFs showing OR2A4/7 (green, white in black and white figure version), and integrin α-6 (red, light grey in black and white figure version) expression (right panel). Nuclei are counterstained with DAPI (blue, dark grey in black and white figure version). All data represented as individual normalization to the vehicle treated control group; mean±SEM.; n=28-49 HFs from n=5-8 independent donors, *p<0.05, ***p<0.001.

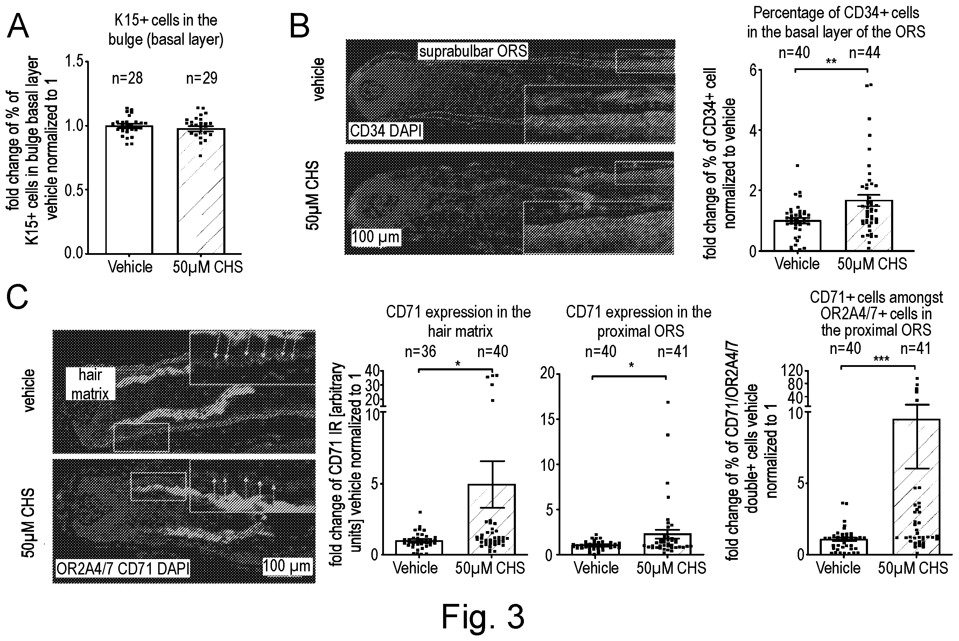

Figure 3. A) illustrates bar graphs showing quantitative analysis of K15+ stem cells in the basal layer of the bulge under vehicle or 50µM CHS treatment. No differences are seen B) Representative images of CD34 (red, light grey in black and white figure version) protein expression in ex vivo HFs (left); bar graphs showing quantitative analysis of CD34+ cells in the basal layer of the outer root sheath (ORS) under vehicle or 50µM CHS treatment (right). C) Representative images of CD71 (red, light grey in black and white figure version) and OR2A4/7 (green, white in black and white figure version) protein co-expression in ex vivo HFs (left); bar graphs showing quantitative analysis of CD71+ cells in the hair matrix and the proximal ORS (middle); bar graph showing percentage of CD71/OR2A4/7 double positive cells in the proximal outer root sheath (right). Nuclei are counterstained with DAPI (blue, dark grey in black and white figure version). All data represented as individual normalization to the vehicle treated control group; mean±SEM.; n=36-44 HFs from n=8 independent donors, *p<0.05, *p<0.01, ***p<0.001.

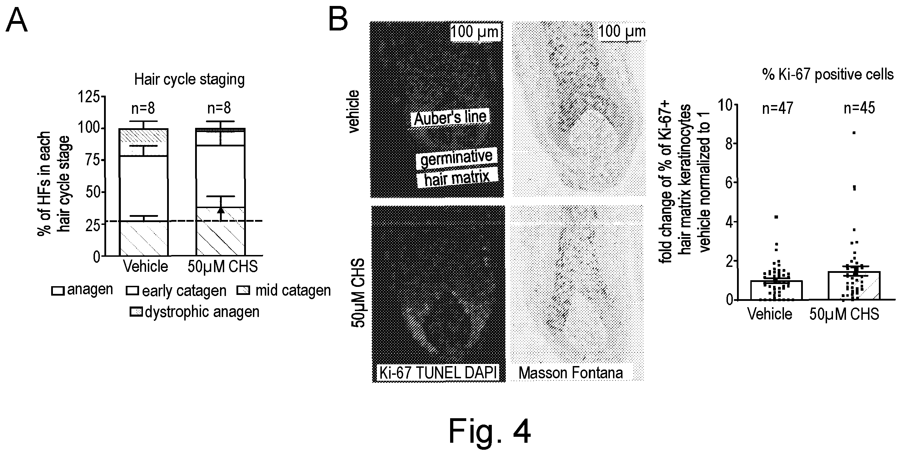

Figure 4. A) illustrates the percentage of HFs in each hair cycle stage under cultivation with vehicle or 50µM CHS. The green arrow (grey in black and white figure version )represents the increase of HFs in anagen under 50µM CHS treatment. n = 8 independent donors. Figure B) illustrates the representative images of Ki-67 (red, light grey in black and white figure version) and TUNEL (green) protein expression in ex vivo HF bulbs, to analyze cell proliferation (left panel); Representative images of Masson Fontana staining in ex vivo HFs bulbs, for melanin staining (right panel); bar graphs showing quantitative analysis of Ki-67 protein expression in hair matrix keratinocytes under vehicle or 50µM CHS treatment. Nuclei are counterstained with DAPI (blue, dark grey in black and white figure version). All data represented as individual normalization to the vehicle treated control group; mean±SEM.; n=45-47 HFs from n=8 independent donors, *p<0.05, ***p<0.001.

### DETAILED DESCRIPTION OF THE INVENTION

In accordance with certain aspects of the invention, the inventors found that a selected olfactory receptor 2A4/7 agonist is suitable for both cosmetic and therapeutic applications in the trichological field.

The Applicant discovered that cyclohexyl salicylate, which is an olfactory receptor 2A4/7 agonist, stimulates the activity of follicle cells and of quiescent hair bulbs of the scalp. These activities activate the life cycle of hair follicles promoting the hair growth and ameliorating the aesthetic aspect of hair.

The compound cyclohexyl salicylate, also known as cyclohexyl 2-hydroxybenzoate, has a CAS No. 25485-88-5.

In accordance with a first aspect the present invention thus provides the cosmetic/ non therapeutical use of an olfactory receptor 2A4/7 agonist for promoting hair growth or preventing or slow down hair loss and/or improving an aesthetic aspect of the hair wherein the olfactory receptor 2A4/7 agonist is cyclohexyl salicylate.

The compound cyclohexyl salicylate is useful in the treatment of a population having aesthetic problems of the hair such as non-physiological hair pigmentation or hair thinning or ruptures of the hair stem.

Typically, the local application of cyclohexyl salicylate on skin or scalp stimulates the physiological growth of hair and the thickening of the hair.

Advantageously, the present invention also provides the cosmetic, non-therapeutic, use of a composition comprising cyclohexyl salicylate and a physiologically acceptable carrier for stimulating the physiological growth of hair.

In accordance with a further aspect the present invention provides an olfactory receptor 2A4/7 agonist for use in the prevention or treatment of a hair growth disorder or affection wherein the olfactory receptor 2A4/7 agonist is cyclohexyl salicylate.

The hair growth disorders or affections which may be treated according to this aspect of the invention include alopecia, especially androgenetic alopecia and alopecia induced by chemotherapy and telogen effluvium, and disorders affecting subjects of male and female gender. The composition as herein described is also suitable for treating or preventing female pattern, baldness.

According to another aspect, a pharmaceutical composition for the medical uses as defined in claim 8 is herein provided. The pharmaceutical composition of the invention is effective in preventing and/or treating forms of baldness or hair thinning as described above.

Example of hair growth disorders treated by the composition include alopecia and telogen effluvium. Typically, these compositions are formulated as pharmaceutical compositions.

The amount of cyclohexyl salicylate contained in the compositions for cosmetic/ pharmaceutical/nutritional uses is such that a prophylactic or therapeutically effective dosage is obtained.

In accordance with certain embodiments the composition for both cosmetic and medical uses may contain cyclohexyl salicylate in an amount of 0.001 to 20% by weight, 0.05 to 15% by weight; 0.1 to 10%, 1-7 by weight.

In certain embodiments, the composition of the invention may further comprise one or more additional ingredients having a trichological activity.

The amount administered and the frequency of administration of the composition will depend on the type and severity of the trichological condition to be treated.

In certain embodiments the compositions for both cosmetic and therapeutical uses further includes additional ingredients.

In some embodiments the composition of the invention for both cosmetic and medical uses comprises a physiologically and/or pharmaceutically acceptable carrier, diluent or excipient.

Typically, the physiologically acceptable carrier of the composition of the invention is an excipient, carrier or diluent suitable for topical application and/or systemic administration. Physiologically and pharmaceutically carriers may be the same carriers.

Within the scope of the present document, the term "carrier" relates to an excipient, carrier, diluent or adjuvant that may be present in the composition of the invention. Any carrier and/or excipient suitable for the form of preparation desired for administration is contemplated in the uses of the vegetal extract or herein described active ingredients present therein.

The term "hair" as described herein includes hair of the scalp, eyelash, eyebrows and the beard.

Typically, a suitable carrier is a physiologically, edible or pharmaceutically acceptable carrier.

The term treatment of "trichological disorder" or "aesthetic aspect" means the treatment of any conditions where there is a change in the physiological conditions of hair, especially a change in an aesthetic aspect of hair. The compositions of the present invention encompass any compositions made by admixing the association of a compound of formula (I) and a physiologically acceptable carrier. Such compositions are suitable for topical application and/or use in a mammal, specifically human beings.

The carrier may take a wide variety of suitable forms depending on the form of preparation desired for the local administration.

Typically, the topical composition is applied onto the skin, and in particular onto the scalp in an effective amount.

The composition for topical application may be in the solid, semi-solid or fluid form. Suitable formulations in the solid form include creams, gels, pastes, ointments.

In other embodiments the formulation for local administration is in the fluid form, for example in the form of lotions, gels, shampoos, suspensions, emulsions.

In the case of formulations in the fluid or semi-fluid form, the compound of formula (I) may be diluted in a carrier in the physiologically acceptable liquid form such as water, alcohol, hydroalcoholic or glyceric solution or mixed with other liquids suitable for local application.

In the form of a solution, suspension or dispersion, the composition of the invention may contain from around 1-99.9% of a liquid such as water optionally mixed with an alcohol. In some embodiments, water is present in an amount of between 5-95%. In other embodiments, water is present in an amount of between 10-90% by weight. A typical composition for topical use is a hydro alcoholic solution containing a compound of formula (I) dissolved therein.

By way of example, the compositions of the invention in liquid form can be prepared by dissolving the active ingredient and the remaining components in alcohol to then reunite the various fractions under agitation. The resulting mixture can then be buffered to reach a pH interval conveniently selected between 5 and 7 so as to be compatible with the pH of the scalp to then be filtered and packaged in suitable containers such as bottles or vials.

In certain embodiments the composition of the invention is in the form of a lotion for external use.

Typically, the active compounds of formula (I) of the pharmaceutical compositions are formulated in dosage units. The dosage unit may contain from 0.1 to 100 mg, from 1 to 10 mg of active ingredient per dosage unit for daily administration.

In some embodiments, the amounts effective for formulation will depend on the severity of the disorder or condition to be treated.

In accordance with certain aspects the invention relates to compositions for topical use which are useful in the stimulation of hair follicles, with a resulting benefit of treating hair growth disorders, such as alopecia, in females in particular.

Methods of treatment of relevant populations are also a feature of the invention.

According to another aspect of the invention, there is provided a method of cosmetic treatment which comprises the application to the scalp of an effective amount of a composition of the type described previously.

For example, for local uses, a cosmetically or pharmaceutically active amount of the composition of the invention is applied directly to the scalp one or more times daily, conveniently for cycles having a duration of 2-3 months alternating with periods of rest.

The following examples are provided merely for illustrating certain aspects of the present invention.

### EXAMPLE 1

| LOTION | |
|---|---|
| component (INCI name) | amount (w/w %) |
| Alcohol denat. | 15.0-35.0 |
| PEG-40 Hydrogenated castor oil | 0.5-6.0 |
| Ethoxydiglycol | 0.1-2.5 |
| Propanediol | 0.1-4.5 |
| Dipropylene Glycol | 0.1-2.5 |
| Cyclohexyl salicylate | 0.1-5.0 |
| Sodium hydroxide 30% solution | 0.001-1.0 |
| Citric acid | 0.003-1.0 |
| Aqua q.s. to 100 g | |

### EXAMPLE 2

| RESTRUCTURING -CONDITIONING MASK | |
|---|---|
| component (INCI name) | amount (w/w %) |
| Propanediol | 0.5-6.5 |
| Ammonium acryloyldimethyltaurateNP copolymer | 1.0-2.0 |
| Cyclohexyl salicylate | 0.3-7.0 |
| Tocopherol | 0.001-0.2 |
| Disiloxane | 1.0-2.0 |
| Parfum | 0.5-1.0 |
| Caprylyl glycol | 0.25-0.75 |
| Phenyl trimethicone | 0.25-0.75 |
| Silicone quaternium-17 | 0.1-0.4 |
| Dimethicone | 0.1-0.4 |
| Dimethiconol | 0.025-0.075 |
| Laureth-4 | 0.1-0.4 |
| Inulin | 0.1-0.4 |
| Tocopheryl acetate | 0.1-0.3 |
| Laureth-23 | 0.1-0.3 |
| Potassium sorbate | 0.05-0.15 |
| Citric acid | 0.04-0.08 |
| Trisodium ethylenediamine Disuccinate | 0.025-0.075 |
| Ethylhexyl methoxycinnamate | 0.025-0.075 |
| Phenoxyethanol | 0.25-0.75 |
| Aqua q.s. to 100 g | |

### EXAMPLE 3

| STYLING GEL | |
|---|---|
| Component (INCI name) | amount (w/w %) |
| Cyclohexyl Salicylate | 0.1-10.0 |
| PEG-40 hydrogenated castor oil | 1.0-3.0 |
| PPG-26-Buteth-26 | 0.1-1.5 |
| Parfum | 0.5-1.5 |
| Polyacrylate-14 | 0.5-1.5 |
| Hydroxypropyl guar | 0.5-1.5 |
| BHA | 0.01-0.1 |
| hydrogenated starch hydrolysate | 0.5-1.5 |
| Disodium EDTA | 0.05-0.15 |
| Polyquaternium-11 | 0.0025-0.025 |
| Aminomethylpropanol | 0.05-6.0 |
| Phenylpropanol | 0.05-1.0 |
| Propanediol | 0.05-3.0 |
| Caprylyl glycol | 0.05-1.0 |
| Sodium benzoate | 0.01-0.1 |
| Sodium hydroxide 30% solution | 0.01-1.0 |
| Citric acid | 0.03-1.0 |
| Hydroxyacetophenone | 0.05-1.0 |
| O-Cymen-5-OL | 0.05-0.1 |
| Aqua q.s. to 100 g | |

### EXAMPLE 4

| FORTIFYING HAIR CONDITIONER | |
|---|---|
| Component (INCI name) | amount (w/w %) |
| Cetyl alcohol | 1.0-7.0 |
| Glyceryl stearate | 0.5-28.0 |
| Cocos nucifera oil | 1.0-5.0 |
| Cetrimonium chloride | 2.5-7.5 |
| Cetearyl alcohol | 0.5-7.5 |
| Behentrimonium methosulfate | 0.1-2.5 |
| PEG-100 stearate | 0.1-3.5 |
| Betaine | 0.1-0.35 |
| Xylitol | 2.0-6.0 |
| Cyclohexyl salicylate | 0.1-10.0 |
| Hydroxyethylcellulose | 1.0-3.0 |
| Isoamyl laurate | 1.0-3.0 |
| Benzyl alcohol | 0.1-1.0 |
| Panthenol | 1.0-3.0 |
| Parfum | 1-2 |
| Bis-Isobutyl PEG/PPG-20/35/Amodimethicone copolymer | 0.5-1.0 |
| Phytantriol | 0.5-1.0 |
| Panthenol | 0.5-1.0 |
| Sodium benzoate | 0.01-0.5 |
| Sodium dehydroacetate | 0.5-1.0 |
| Cetyl ethylhexanoate | 0.5-1.0 |
| Butylene glycol | 0.5-1.0 |
| Disodium EDTA | 0.2-0.6 |
| Polysorbate 80 | 0.2-0.6 |
| Hydroxypropyltrimonium Hyaluronate | 0.1-0.6 |
| Dehydroacetic acid | 0.1-0.3 |
| Gluconolactone | 0.05-0.15 |
| Sodium hydroxide 30% solution | 0.01-0.5 |
| Lactic acid 80% | 0.02-1.0 |
| Aqua q.s. to 100 g | |

### EXAMPLE 5

| REVITALIZING SHAMPOO | |
|---|---|
| Component (INCI name) | amount(w/w %) |
| Magnesium laureth sulfate | 5.0-10.0 |
| Sodium lauroyl sarcosinate | 2.0-3.0 |
| Cyclohexyl salicylate | 0.1-10.0 |
| Disodium laureth sulfosuccinate | 1.5-2.5 |
| PEG-200 hydrogenated glyceryl palmate | 1.0-2.0 |
| Cocamide MIPA | 0.5-1.5 |
| Parfum | 0.5-1 |
| Glycol distearate | 0.5-1 |
| PEG-7 glyceryl cocoate | 0.25-0.75 |
| Betaine | 0.25-0.75 |
| Lauryl methyl gluceth-10 hydroxypropyldimonium chloride | 0.25-0.75 |
| Laureth-7 | 0.25-0.75 |
| Polyquaternium-10 | 0.25-0.75 |
| Hydroxyacetophenone | 0.05-1.0 |
| O-Cymen-5-OL | 0.05-0.1 |
| Potassium undecylenoyl hydrolyzed wheat protein | 0.2-0.4 |
| Panthenol | 0.1-0.3 |
| Disodium EDTA | 0.1-0.3 |
| Phenyl trimethicone | 0.05-0.15 |
| Silicone quaternium-17 | 0.05-0.12 |
| Laureth-4 | 0.05-0.12 |
| Laureth-23 | 0.025-0.075 |
| Sodium cocoamphoacetate | 0.025-0.075 |
| BHA | 0.005-0.015 |
| Sodium benzoate | 0.1-0.5 |
| Potassium sorbate | 0.05-0.5 |
| Sodium hydroxide 30% solution | 0.01-0.5 |
| Lactic acid 80% | 0.02-1.0 |
| Aqua q.s. to 100 g | |

### EXAMPLE 6

| MOUSSE | |
|---|---|
| Component (INCI name) | amount (w/w %) |
| Alcohol | 10-20 |
| Cyclohexyl salicylate | 0.1-1.2 |
| PEG-40 hydrogenated castor oil | 1-2 |
| Glycerin | 1-1.5 |
| Sodium olivamphoacetate | 0.5-1.5 |
| Parfum | 0.5-1 |
| Tocopherol | 0.05-0.15 |
| Disodium EDTA | 0.025-0.075 |
| Polyquaternium-16 | 0.025-0.075 |
| Potassium metabisulfite | 0.01-0.03 |
| Sodium benzoate | 0.1-0.5 |
| Lactic acid 80% | 0.02-1.0 |
| Aqua q.s. to 100 g | |

### EXAMPLE 7

### Rationale:

Hair Follicle (HF) disorders and aging result in hair thinning and hair loss. Affected individuals suffer from severe psychological stress. Therefore, developing topically applicable formulations that can serve as alternative or as therapeutic addition for drugs to improve hair density, is of great importance.

Human keratinocytes express an ORs, newly identified as OR2A4/7, which showed similarities but also differences concerning their physiological effects following their activation by specific agonists (Tsai et al., Exp Dermatol. 2017). In the literature, OR2A4/7 expression has been reported in the epidermis of human skin, as well as in primary and immortalized human keratinocytes (HaCaT) (Tsai et al., Exp Dermatol. 2017).

In this study inventors investigated the functional role of the olfactory receptor OR2A4/7 in normal human melanocytes following stimulation by a specific ligand, cyclohexyl salicylate (CHS).

The inventors focused on OR2A4/7, which promotes epidermal keratinocyte proliferation and differentiation. OR2A4/7 has been de-orphaned, since CHS was identified as a specific agonist in a screening approach (Tsai et al., Exp Dermatol. 2017). In particular, OR2A4/7 is functionally expressed and active in human melanocytes, because CHS elicited transient intracellular Ca2+ signals.

For the first time the inventors show, OR2A4/7 protein expression in the infundibulum, and isthmus, (suprabasal ORS) of HF in normal human skin in situ.

### MATERIALS AND METHODS:

### 1.1 In situ hybridization and immunofluorescence staining on fresh scalp skin, immediately frozen after extraction, to characterize OR2A4/7 mRNA and protein expression in the HF

For in situ hybridization normal human full-thickness scalp skin (temporal/occipital) with HFs were used. The samples were left untreated and directly frozen on D0. Frozen samples were sectioned with a cryostat (Leica) and 7 µm sections were collected. The skin sample was carefully orientated to obtain full-length HF sections. Consecutive sections of each full-length HF were collected and slides stored at -80°C (Haslam, I.S. , Hardman, J.A. and Paus, R. Topically applied nicotinamide inhibits human hair follicle growth ex vivo. J. Invest. Dermatol. 138, 1420-1422 (2018)). Sections were then postfixed by immersion in 4% PFA and then washed in PBS (pH7.4) for 15min.

After washing steps the sections were in situ hybridized. Custom-designed probes from RNAScope (Advanced Cell Diagnostics Srl, Milan, Italy) were used.

Full-length human HF *ex vivo* organ cultures from follicular units (occipital) were used for immunofluorescence staining. The organ culture was left untreated, directly frozen on day of isolation (D0). Frozen samples were sectioned with a cryostat (Leica) and 7 µm sections were collected. The skin sample was carefully orientated to obtain full-length HF sections. Consecutive sections of each full-length HF were collected and slides stored at -80°C (Haslam, I.S., Hardman, J.A. and Paus, R. Topically applied nicotinamide inhibits human hair follicle growth ex vivo. J. Invest. Dermatol. 138, 1420-1422 (2018)). Sections obtained from the organ culture were stained using a propetary antibody and staining protocol. Sections without primary antibody were used as negative control. Counterstaining to visualize nuclei was performed with 4',6-diamidino-2-phenylindole (DAPI) (Sigma-Aldrich Biochemie GmbH, Hamburg, Germany).

Images of the evaluation areas were taken at original magnifications of 100× or200× with Keyence-Biozero 8100 and 9100 microscopes (Keyence Corporation, Osaka, Japan).

Staining intensity was evaluated in well-defined reference areas by quantitative (immuno-)histomorphometry (Fischer TW, et al. Differential effects of caffeine on hair shaft elongation, matrix and outer root sheath keratinocyte proliferation, and transforming growth factor-β2/insulin-like growth factor-1-mediated regulation of the hair cycle in male and female human hair follicles in vitro. Br. J. Dermatol. 2014;171:1031-1043. doi: 10.1111/bjd.13114.; Samuelov L, et al. P-cadherin regulates human hair growth and cycling via canonical Wnt signaling and transforming growth factor-β2. J. Invest. Dermatol. 2012;132:2332-2341. doi: 10.1038/jid.2012.171.) using NIH ImageJ software (NIH, Bethesda, MD, USA).

### 1.2Organ cultures of microdissected human HFs in the presence of the cosmetic OR2A4/7 agonist, cyclohexyl salicylate (CHS), and effects on hair follicle stem cell and progeny number, by quantitative (immuno)histomorphometry

Temporal and occipital human scalp skin was obtained from healthy donors (38-69 years old) undergoing routine face-lift surgery. Scalp skin samples were processed for HF microdissection (Edelkamp J, Gherardini J, Bertolini M, Methods to study human hair follicle growth ex vivo: human microdissected hair follicle and human full thickness skin organ culture, Methods Mol. Biol. 2154 (2020) 105-119). Microdissected human scalp HFs were cultured at 37 °C with 5% CO2 in a minimal media of William's E media (WEM, Gibco, Life Technologies) supplemented with 2 mM of L-glutamine (Gibco), 10 ng/ml hydrocortisone (Sigma-Aldrich), 10 µg/ml insulin (Sigma-Aldrich), and 1% penicillin/streptomycin mix (Gibco)25,26,62. After microdissection, the HFs were first incubated in WEM for 24 h for re-equilibration. HFs after quality control (fully pigmented and presence in anagen VI phase) were randomly allocated to the different experimental groups.

After 24 h, WEM medium was replaced and HFs were treated with vehicle or 50µM CHS for 6 days for (immuno)histomorphometry.

OCT-embedded samples were sectioned (6 µm thickness for HF and 7 µm thickness for skin) with a Leica cryostat. Sections obtained from the organ culture were stained using OR2A4/7 and integrin α-6 antibodies. A double-staining, and OR2A4/7 co-localizes with CD71 only in selected hair follicle compartments, i.e suprabulbar ORS, and hair matrix was also performed. Sections without primary antibody were used as negative control. Counterstaining to visualize nuclei was performed with 4',6-diamidino-2-phenylindole (DAPI) (Sigma-Aldrich Biochemie GmbH, Hamburg, Germany).

Images of the evaluation areas were taken at original magnifications of 100× or200× with Keyence-Biozero 8100 and 9100 microscopes (Keyence Corporation, Osaka, Japan).

Staining intensity was evaluated in well-defined reference areas by quantitative (immuno-)histomorphometry (Fischer TW, et al. Differential effects of caffeine on hair shaft elongation, matrix and outer root sheath keratinocyte proliferation, and transforming growth factor-β2/insulin-like growth factor-1-mediated regulation of the hair cycle in male and female human hair follicles in vitro. Br. J. Dermatol. 2014;171:1031-1043. doi: 10.1111/bjd.13114.; Samuelov L, et al. P-cadherin regulates human hair growth and cycling via canonical Wnt signaling and transforming growth factor-β2. J. Invest. Dermatol. 2012;132:2332-2341. doi: 10.1038/jid.2012.171.) using NIH ImageJ software (NIH, Bethesda, MD, USA).

### 1.3Organ cultures of microdissected HFs in the presence of CHS and effect on hair cycle and hair matrix keratinocyte proliferation by quantitative (immuno)histomorphometry and Masson Fontana staining

Temporal and occipital human scalp skin was obtained from healthy donors (38-69 years old) undergoing routine face-lift surgery. Scalp skin samples were processed for HF microdissection (Edelkamp J, Gherardini J, Bertolini M, Methods to study human hair follicle growth ex vivo: human microdissected hair follicle and human full thickness skin organ culture, Methods Mol. Biol. 2154 (2020) 105-119). Microdissected human scalp HFs were cultured at 37 °C with 5% CO2 in a minimal media of William's E media (WEM, Gibco, Life Technologies) supplemented with 2 mM of L-glutamine (Gibco), 10 ng/ml hydrocortisone (Sigma-Aldrich), 10 µg/ml insulin (Sigma-Aldrich), and 1% penicillin/streptomycin mix (Gibco)25,26,62. After microdissection, the HFs were first incubated in WEM for 24 h for re-equilibration. HFs after quality control (fully pigmented and presence in anagen VI phase) were randomly allocated to the different experimental groups.

After 24 h, WEM medium was replaced and HFs were treated with vehicle or 50µM CHS for 6 days for (immuno-)histomorphometry.

For histochemical visualization of melanin, Masson-Fontana staining was performed on frozen sections. Melanin was stained as brown dots (Kloepper JE, et al. Methods in hair research: how to objectively distinguish between anagen and catagen in human hair follicle organ culture. Exp. Dermatol. 2010;19:305-312. doi: 10.1111/j.1600-0625.2009.00939.x.).

OCT-embedded samples were sectioned (6 µm thickness for HF and 7 µm thickness for skin) with a Leica cryostat. To stain apoptotic and proliferating cells, we used the apoptag kit (Merck Milipore) following the manufacturer's protocol followed by Ki-67 staining (Langan EA, et al. Human hair follicle organ culture: theory, application and perpsectives. Exp. Dermatol. 2015;24:903-911. doi: 10.1111/exd.12836; Kloepper JE, et al. Methods in hair research: how to objectively distinguish between anagen and catagen in human hair follicle organ culture. Exp. Dermatol. 2010;19:305-312. doi: 10.1111 /j.1600-0625.2009.00939.x; Ahn SY, et al. Effect of IGF-I on hair growth is related to the anti-apoptotic effect of IGF-I and up-regulation of PDGF-A and PDGF-B. Ann. Dermatol. 2012;24:26-31. doi: 10.5021/ad.2012.24.1.26.; PurbaTS, et al. A primer for studying cell cycle dynamics of the human hair follicle. Exp. Dermatol. 2016;25:663-668. doi: 10.1111/exd.13046). Primary antibody was incubated overnight (Ki-67, M7240 Clone: MIB-1, DAKO, 1/20) after the TdT-enzyme step. The secondary antibody was incubated for 45 min at RT after the fluorescent-labeled anti-Digoxigenin step of the apoptag kit. Counterstaning with DAPI was performed to visualize nuclei. Negative controls were performed by omitting the primary antibody. Images were taken using a Keyence fluorescence microscope BZ9100 (Osaka, Japan) maintaining a constant set exposure time throughout imaging for further analysis. HFs were microscopically evaluated for the hair cycle staging analysis using Masson-Fontana histochemistry and Ki-67/TUNEL immunostainings (Langan EA, et al. Human hair follicle organ culture: theory, application and perpsectives. Exp. Dermatol. 2015;24:903-911. doi: 10.1111/exd.12836; KloepperJE, etal. Methods in hair research: how to objectively distinguish between anagen and catagen in human hair follicle organ culture. Exp. Dermatol. 2010;19:305-312. doi: 10.1111/j.1600-0625.2009.00939.x;). The HCS was also measured (Langan EA, et al. Human hair follicle organ culture: theory, application and perpsectives. Exp. Dermatol. 2015;24:903-911. doi: 10.1111/exd.12836; Fischer TW, etal. Differential effects of caffeine on hair shaft elongation, matrix and outer root sheath keratinocyte proliferation, and transforming growth factor-β2/insulin-like growth factor-1-mediated regulation of the hair cycle in male and female human hair follicles in vitro. Br. J. Dermatol. 2014;171:1031-1043. doi: 10.1111/bjd.13114.) which consists of assigning an arbitrary unit for each stage of the hair cycle (Anagen VI = 100; Early catagen = 200; Mid-catagen = 300; and Late catagen = 400). After having classified each HF according to its hair cycle stage, following the previously defined objective classification criteria for organ-cultured human HFs (Kloepper JE, et al. Methods in hair research: how to objectively distinguish between anagen and catagen in human hair follicle organ culture. Exp. Dermatol. 2010;19:305-312. doi: 10.1111/j.1600-0625.2009.00939.x), for each experimental condition, the mean HCS was calculated. The closer the mean is to 100, the higher is the number of anagen VI HFs in a given group. The HCS provides a global read-out parameter that looks at all HFs in a given experimental group and synthesizes them into a single number, which reflects how close the majority of HFs is to either anagen VI or catagen and also permits statistical analysis that it is not possible with hair cycle staging. Therefore, hair cycle staging and the HCS are independent read-out parameters that complement each other. Staining intensity was evaluated in well-defined reference areas by quantitative (immuno-)histomorphometry (Fischer TW, et al. Differential effects of caffeine on hair shaft elongation, matrix and outer root sheath keratinocyte proliferation, and transforming growth factor-β2/insulin-like growth factor-1-mediated regulation of the hair cycle in male and female human hair follicles in vitro. Br. J. Dermatol. 2014;171:1031-1043. doi: 10.1111/bjd.13114.; Samuelov L, et al. P-cadherin regulates human hair growth and cycling via canonical Wnt signaling and transforming growth factor-β2. J. Invest. Dermatol. 2012;132:2332-2341. doi: 10.1038/jid.2012.171.) using NIH ImageJ software (NIH, Bethesda, MD, USA).

### RESULTS:

As reported by Tsai et al., Exp Dermatol, 2017, OR2A4/7 protein is expressed in epidermis in normal skin in situ, confirming the specificity of the inventors' immunostaining. The inventors successfully established RNA in situ hybridization for OR2A4/7 and showed OR2A4/7 RNA expression in the whole epidermis, hair bulb (HM, ORS, and IRS), and ORS keratinocytes within the bulge area of HF in normal skin *in situ* (Figure 1).

Indeed, the inventors detected ubiquitous expression of OR2A4/7 mRNA in the HF epithelium, namely the outer root sheath (ORS) and hair matrix (HM) by in situ hybridization (Figure 1a).

Analysis of freshly embedded human scalp skin revealed that expression of OR2A4/7 protein was mainly restricted to the infundibulum. However, during HF organ culture (OC) OR2A4/7 protein became widely expressed in the ORS and HM, suggesting up-regulation of intrafollicular OR2A4/7 expression under tissue stress conditions (Figure 1b).

OR2A4/7 expression increases under organ culture conditions, and CHS treatment results in receptor downregulation in the HF bulb ex *vivo* (Figure 2).

Therefore OR2A4/7 stimulation significantly increases HF stem cell progeny ex vivo (Figure 3). While CHS did not seem to impact on K15+ bulge stem cells (Figure 3a), expression of CD34+ cells, their immediate progeny, was significantly increased in the suprabulbar ORS (Figure 3b). The same held true for the % of CD71+ transit amplifying cells (thought to derive from CD34+ cells) in the HM and suprabulbar ORS (Figure 3c).

OR2A4/7 stimulation also prolongs anagen (Figure 4a) and induces hair matrix keratinocyte proliferation in HFs ex vivo (Figure 4b).

## Claims

1. A non-therapeutic, cosmetic use of an olfactory receptor 2A4/7 agonist for improving an aesthetic aspect of the hair, wherein the olfactory receptor 2A4/7 agonist is cyclohexyl salicylate.

2. The non-therapeutic, cosmetic use according to claim 1 wherein the aesthetic aspect is hair thinning.

3. A non-therapeutic, cosmetic use of a composition comprising an olfactory receptor 2A4/7 agonist and a physiologically acceptable carrier, for improving an aesthetic aspect of the hair, wherein the olfactory receptor 2A4/7 agonist is cyclohexyl salicylate.

4. The non-therapeutic, cosmetic use according to claim 3, wherein the aesthetic aspect is hair thinning.

5. The non-therapeutic, cosmetic use according to claim 3 or 4 wherein the composition is for topical application.

6. An olfactory receptor 2A4/7 agonist for use in the prevention or treatment of a hair growth disorder wherein the olfactory receptor 2A4/7 agonist is cyclohexyl salicylate.

7. The olfactory receptor 2A4/7 agonist for use according to claim 6 wherein the hair growth disorder is selected from the group consisting of alopecia, alopecia areata, telogenicum effluvium, and androgenetic alopecia and alopecia areata induced by chemotherapy.

8. A pharmaceutical composition comprising an olfactory receptor2A4/7 agonist and a pharmaceutically acceptable carrier for use in the prevention or treatment of a hair growth disorder wherein the olfactory receptor2A4/7 agonist is cyclohexyl salicylate.

9. The pharmaceutical composition for use according to claim 8 wherein the composition is for local/topical application.

10. The pharmaceutical composition for use according to claim 8 or 9 wherein the hair growth disorder is selected from the group consisting of alopecia, alopecia areata, telogenic effluvium, and androgenetic alopecia and alopecia induced by chemotherapy.

## Patentansprüche

1. Nicht-therapeutische, kosmetische Verwendung eines olfaktorischen Rezeptor-2A4/7-Agonisten zur Verbesserung des ästhetischen Aspekts des Haars, wobei der olfaktorische Rezeptor-2A4/7-Agonist Cyclohexylsalicylat ist.

2. Nicht-therapeutische, kosmetische Verwendung nach Anspruch 1, wobei der ästhetische Aspekt schütteres Haar ist.

3. Nicht-therapeutische, kosmetische Verwendung einer Zusammensetzung, die einen olfaktorischen Rezeptor-2A4/7-Agonisten und einen physiologisch verträglichen Träger umfasst, zur Verbesserung des ästhetischen Aspekts des Haars, wobei der olfaktorische Rezeptor-2A4/7-Agonist Cyclohexylsalicylat ist.

4. Nicht-therapeutische, kosmetische Verwendung nach Anspruch 3, wobei der ästhetische Aspekt schütteres Haar ist.

5. Nicht-therapeutische, kosmetische Verwendung nach Anspruch 3 oder 4, wobei die Zusammensetzung zur topischen Anwendung bestimmt ist.

6. Olfaktorischer Rezeptor-2A4/7-Agonist zur Verwendung bei der Vorbeugung oder Behandlung einer Haarwuchsstörung, wobei der olfaktorische Rezeptor-2A4/7-Agonist Cyclohexylsalicylat ist.

7. Olfaktorischer Rezeptor-2A4/7-Agonist zur Verwendung nach Anspruch 6, wobei die Haarwuchsstörung ausgewählt ist aus der Gruppe, bestehend aus Alopezie, Alopecia aerata, Telogen Effluvium und androgenetischer Alopezie und durch Chemotherapie induzierter Alopecia aerata.

8. Pharmazeutische Zusammensetzung, die einen olfaktorischen Rezeptor-2A4/7-Agonisten und einen pharmazeutisch verträglichen Träger umfasst, zur Verwendung bei der Vorbeugung oder Behandlung einer Haarwuchsstörung, wobei der olfaktorische Rezeptor-2A4/7-Agonist Cyclohexylsalicylat ist.

9. Pharmazeutische Zusammensetzung zur Verwendung nach Anspruch 8, wobei die Zusammensetzung zur lokalen/topischen Anwendung bestimmt ist.

10. Pharmazeutische Zusammensetzung zur Verwendung nach Anspruch 8 oder 9, wobei die Wuchsstörung ausgewählt ist aus der Gruppe, bestehend aus Alopezie, Alopecia aerata, Telogen Effluvium und androgenetischer Alopezie und durch Chemotherapie induzierter Alopecia aerata.

## Revendications

1. Utilisation cosmétique non thérapeutique d'un agoniste du récepteur olfactif 2A4/7 pour améliorer un aspect esthétique des cheveux, dans laquelle l'agoniste du récepteur olfactif 2A4/7 est le salicylate de cyclohexyle.

2. Utilisation cosmétique non thérapeutique selon la revendication 1, dans laquelle l'aspect esthétique est l'amincissement des cheveux.

3. Utilisation cosmétique non thérapeutique d'une composition comprenant un agoniste du récepteur olfactif 2A4/7 et un véhicule physiologiquement acceptable, pour améliorer un aspect esthétique des cheveux, dans laquelle l'agoniste du récepteur olfactif 2A4/7 est le salicylate de cyclohexyle.

4. Utilisation cosmétique non thérapeutique selon la revendication 3, dans laquelle l'aspect esthétique est l'amincissement des cheveux.

5. Utilisation cosmétique non thérapeutique selon la revendication 3 ou 4, dans laquelle la composition est destinée à une application topique.

6. Agoniste du récepteur olfactif 2A4/7 destiné à être utilisé dans la prévention ou le traitement d'un trouble de la croissance des cheveux dans lequel l'agoniste du récepteur olfactif 2A4/7 est le salicylate de cyclohexyle.

7. Agoniste du récepteur olfactif 2A4/7 à utiliser selon la revendication 6, dans lequel le trouble de la croissance des cheveux est choisi dans le groupe constitué par l'alopécie, l'alopécie aerata, l'effluvium télogène et l'alopécie androgénétique et l'alopécie aerata induite par la chimiothérapie.

8. Composition pharmaceutique comprenant un agoniste du récepteur olfactif 2A4/7 et un véhicule pharmaceutiquement acceptable pour une utilisation dans la prévention ou le traitement d'un trouble de la croissance des cheveux, dans laquelle l'agoniste du récepteur olfactif 2A4/7 est le salicylate de cyclohexyle.

9. Composition pharmaceutique à utiliser selon la revendication 8, dans laquelle la composition est destinée à une application locale/topique.

10. Composition pharmaceutique à utiliser selon la revendication 8 ou 9, dans laquelle le trouble de la croissance est choisi dans le groupe constitué par l'alopécie, l'alopécie aerata, l'effluvium télogène et l'alopécie androgénétique et l'alopécie aerata induite par la chimiothérapie.
